# EUROPEAN PATENT APPLICATION

(11) **EP 4 023 122 A1**
(43) Date of publication of application: **06.07.2022**
(21) Application number: 21870527.5
(22) Date of filing: 31.08.2021
(51) Int. Cl.: A47K 10/16

(54) **TISSUE PAPER AND TISSUE PAPER PRODUCTS**

(30) Priority: 30.09.2020 JP 2020165556
(71) Applicant: DAIO PAPER CORPORATION, Shikokuchuo-shi Ehime 799-0492 (JP)
(72) Inventor: MANABE, Sadanao, Shikokuchuo-shi, Ehime 799-0113 (JP); OCHI, Ryoichi, Shikokuchuo-shi, Ehime 799-0113 (JP); SHIINOKI, Yuusuke, Fuji-shi, Shizuoka 419-0202 (JP)
(74) Representative: Williams Powell
(86) International application number: PCT/JP2021/031969
(87) International publication number: WO 2022/070733

(57) **Abstract**

To provide a three-ply or four-ply tissue paper that reduces a sticky feeling of an outer surface while securing necessary softness, and a method for manufacturing the same.

A tissue paper including three stacked plies or four stacked plies,
wherein each ply contains an aqueous moisturizer exhibiting hygroscopicity, and
the content of the aqueous moisturizer between the plies is set such that the dry moisturizer content percentage of each ply is within a range of 92.0 to 108.0 when the dry moisturizer content percentage of a product is 100.

## Description

### Technical Field

The present invention relates to a tissue paper, particularly to a tissue paper and a tissue paper product to which a moisturizer is applied.

### Background Art

A two-ply tissue paper is mainly used. However, in recent years, demand for a multi-ply tissue paper that provides a thick feeling, such as a three-ply or four-ply tissue paper, is also increasing.

Such a multi-ply tissue paper is often formed into a product belonging to a high-grade type with a high product price, and the tissue paper of such a product group is required to have "fluffy bulkiness", "surface smoothness", and "softness" particularly corresponding to the number of plies.

In the case of the multi-ply structure, when the basis weight of each ply is increased, the paper thickness can be easily increased by the sum of the plies, and "fluffy bulkiness" can be exhibited.

However, when the basis weight of each ply is increased as described above, it is important to exhibit "fluffy bulkiness" while securing softness and a smooth quality.

Various types of tissue papers are commercially available, and one field thereof is called a moisturizing tissue or a lotion tissue containing an aqueous moisturizer.

A conventional moisturizing tissue and lotion tissue are manufactured so as to have a soft and smooth quality such that the skin does not become red and does not become painful even when used repeatedly for a person who frequently blows his or her nose with the tissue paper due to pollinosis, cold, or the like.

A use form of a tissue paper not a little varies depending on a country. This is considered to be based on a difference in life or culture.

As the moisturizing tissue or the lotion tissue in Japan, a two-ply tissue paper is mainly used, but for example, in China, a three-ply tissue paper is mainly used.

### Citation List

### Patent Literature

Patent Literature 1: JP 2018-171254 A

### Summary of Invention

### Technical Problem

In a conventional moisturizing tissue or lotion tissue including three stacked plies or manufacture thereof, a moisturizer is applied only to both outer layer sheets, and the moisturizer is not applied to an intermediate layer sheet.

In the conventional moisturizing or lotion tissue, it is necessary to apply a certain amount of the moisturizer in order for a user to feel softness at the time of use.

However, on the other hand, when the application amount of the moisturizer is large, stickiness is caused on an outer surface of the outer layer sheet, which is not preferable.

Therefore, the present invention is to provide a three-ply or four-ply tissue paper that reduces a sticky feeling of an outer surface while securing necessary softness, and a method for manufacturing the same.

### Solution to Problem

A tissue paper according to the present invention that has solved the above problems is a tissue paper including three stacked plies or four stacked plies,
wherein each ply contains an aqueous moisturizer exhibiting hygroscopicity, and
the content of the aqueous moisturizer between the plies is set such that the dry moisturizer content percentage of each ply is within a range of 92.0 to 108.0 when the dry moisturizer content percentage of a product is 100.

A tissue paper product according to the present invention is a tissue paper product in which a tissue paper including three stacked plies or four stacked plies is housed in a housing,
wherein each ply contains an aqueous moisturizer exhibiting hygroscopicity, and
the content of the aqueous moisturizer between the plies is set such that the dry moisturizer content percentage of each ply is within a range of 92.0 to 108.0 when the dry moisturizer content percentage of a product is 100.

### Advantageous Effects of Invention

According to the present invention, it is possible to obtain a three-ply or four-ply tissue paper that reduces a sticky feeling of an outer surface while securing necessary softness.

### Brief Description of Drawings

Fig. 1 is a schematic diagram of a chemical solution application form.
Fig. 2 is an explanatory diagram of a rotary interfolder.
Fig. 3 is a schematic diagram of a stacked form.
Fig. 4 is an explanatory diagram of an embodiment of three plies.
Fig. 5 is an explanatory diagram of an embodiment of four plies.
Fig. 6 is an explanatory diagram of an embodiment of conventional three plies.
Fig. 7 is an explanatory diagram of an embodiment of conventionally assumed four plies.
Fig. 8 is a photograph of an example of a chemical solution applied surface.
Fig. 9 is a photograph of an example of a chemical solution non-applied surface.

### Description of Embodiments

Hereinafter, the present invention will be further described with reference to an embodiment of the present invention.

A tissue paper of the present embodiment includes three stacked plies or four stacked plies.

The tissue paper is not a two-ply paper but a three-ply or four-ply paper, and therefore provides "fluffy bulkiness".

To each ply, an aqueous moisturizer (hereinafter, also referred to as "chemical solution", which is synonymous with aqueous moisturizer) exhibiting hygroscopicity is applied, and each ply contains the moisturizer. As the aqueous moisturizer exhibiting hygroscopicity, it is possible to use a moisturizer containing a polyol as a main component, particularly a moisturizer containing glycerin as a main component, that is, a moisturizer containing more than 50% by mass of glycerin, desirably containing 70% by mass or more of glycerin. Glycerin exhibits hygroscopicity in addition to a moisturizing property.

Liquid paraffin can be contained in order to improve surface properties, particularly smoothness. If necessary, 1,3 propanediol can be contained in an amount of 6.1% by mass or more and 12.6% by mass or less.

The tissue paper may contain a known auxiliary agent in addition to the moisturizer such as glycerin. Examples of the auxiliary agent include a moisturizing auxiliary component such as sorbitol, a hydrophilic polymer gelling agent, a surfactant, and a flexibility improver for enhancing moisture retention in the tissue paper, an oil component for aiding exhibition of smoothness, such as the liquid paraffin, an emulsifier for stabilizing the moisturizer and improving applicability, an antiseptic agent, and an antifoaming agent. Note that the blending amount of a component such as the moisturizing auxiliary component or the hydrophilic polymer gelling agent for enhancing moisture retention is set to such an extent that the blending amount does not excessively affect "fluffy bulkiness", "softness", or "surface smoothness". Specifically, the blending amount is 1.0% by mass or less, preferably 0.6% by mass or less, and more preferably 0.5% by mass or less.

It is desirable that the basis weight of one ply of the tissue paper of the present embodiment is 15.0 to 22.5 g/m², and particularly 15.5 to 20.5 g/m². When the basis weight of each layer is within this range, "softness" and "fluffy bulkiness" are remarkably exhibited.

Since paper tends to be hard when the basis weight is high and to be soft when the basis weight is low, it is considered that the basis weight has a large influence on "softness". When the tissue paper is a three-ply or four-ply paper under this basis weight, the tissue paper remarkably provides "fluffy bulkiness". Note that the basis weight is a value measured on the basis of JIS P 8124 (1998).

The aqueous moisturizer is substantially uniformly contained in each ply. That is, the dry moisturizer (chemical solution) content percentage of each ply is within a range of 92.0 to 108.0 when the dry moisturizer (chemical solution) content percentage of a product is 100. It is desirable that the dry moisturizer (chemical solution) content percentage of each ply is within a range of 95.0 to 104.0.

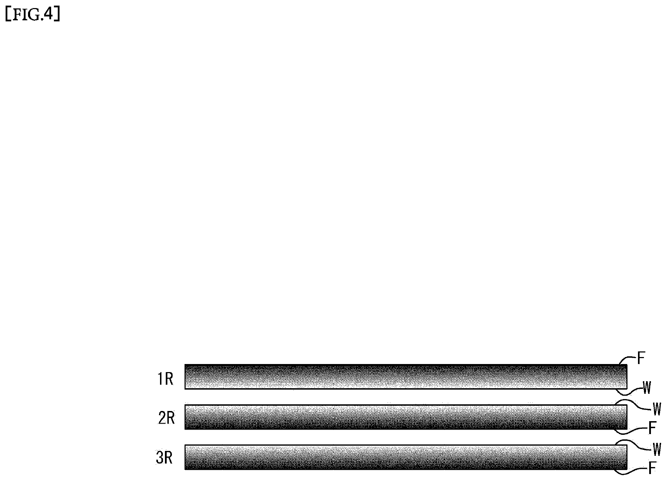

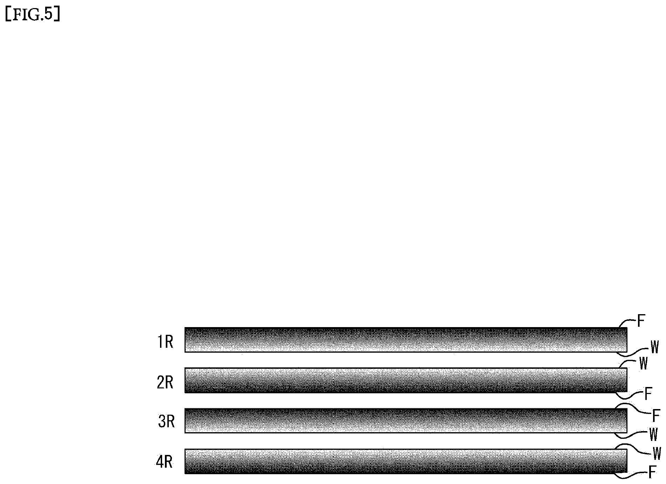

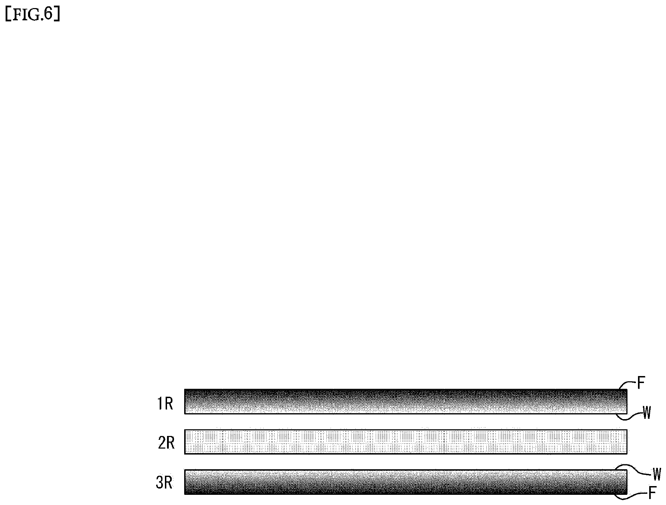

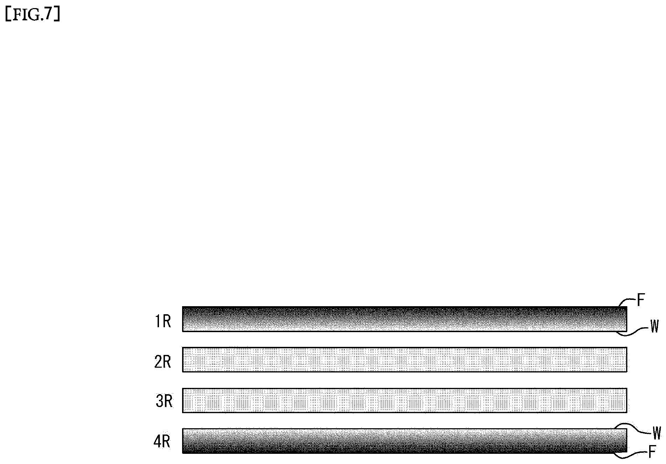

Fig. 4 illustrates an embodiment of three layers (1R, 2R, and 3R), Fig. 5 illustrates an embodiment of four layers (1R, 2R, 3R, and 4R), Fig. 6 illustrates a conventional embodiment of three layers (1R, 2R, and 3R), and Fig. 7 illustrates a conventional embodiment of three layers (1R, 2R, and 3R).

A moisturizer applied surface F and a moisturizer non-applied surface W are illustrated. Since there is not a little gradient of the concentration of the contained moisturizer in a thickness direction from the applied surface F toward the non-applied surface W, the concentration gradient is illustrated in gradation.

As described above, in a conventional moisturizing tissue (also referred to as a lotion tissue), it is necessary to apply a certain amount of the moisturizer in order for a user to feel softness at the time of use. Therefore, since the moisturizer is not applied to the intermediate layer (2R in Fig. 6, and 2R and 3R in Fig. 7) in the conventional embodiments illustrated in Figs. 6 and 7, the application amount of the moisturizer to the outer layer sheet tends to be large, and an outer surface of the outer layer sheet is sticky, which is not preferable.

On the other hand, in the tissue paper of the embodiment, unlike the conventional multilayer tissue paper, the aqueous moisturizer is also applied to the intermediate layer (intermediate ply), that is, (2R in Fig. 4, 2R and 3R in Fig. 5).

An advantage of this configuration is that the intermediate layer (intermediate ply) is soft to make the entire stacked sheets soft because the intermediate layer (intermediate ply) also contains the aqueous moisturizer.

As a result, the amount of the moisturizer contained in the outer layer sheet can be reduced, and the sticky feeling caused by the moisturizer on an outer surface of the outer layer sheet (exposed surface of the stacked sheets) can be eliminated or suppressed.

Note that this advantage is exhibited substantially irrespective of the basis weight of each ply and the content of the moisturizer in each ply as long as the dry moisturizer (chemical solution) content percentage of each ply is within the above range when the dry moisturizer (chemical solution) content percentage of a product is 100.

The aqueous moisturizer can be contained also in the intermediate layer (intermediate ply) by directly applying the moisturizer also to the intermediate layer in addition to the outer layer.

For example, when the moisturizer is applied to the outer layer sheet of the stacked sheets, the moisturizer on the outer layer sheet behaves so as to permeate and diffuse to the entire stacked sheets from the applied surface over time. However, when an external pressure due to calendering or the like is applied to the stacked sheets, a part of the moisturizer applied to the outer layer sheet of the stacked sheets is transferred to an adjacent sheet. However, when the application amount of the moisturizer is small, the amount of the moisturizer transferred to an adjacent sheet is small.

On the other hand, when the applied amount of the moisturizer is large, although the amount of the moisturizer transferred to an adjacent sheet is large, there is a limit to the transfer, and a clear difference is observed between the content of the moisturizer in the outer layer sheet to which the moisturizer is applied and the content of the moisturizer in the adjacent sheet.

Therefore, in order to contain a moisturizer necessary for securing softness also in the intermediate layer (intermediate ply), it is necessary to directly apply the moisturizer also to the intermediate layer in addition to the outer layer.

Conversely, when an excessive amount of the moisturizer is applied to the outer layer sheet in order to transfer the moisturizer to the intermediate layer and to secure softness, a large amount of the moisturizer remains on the outer surface (exposed surface) of the outer layer sheet, and causes stickiness.

However, by directly applying the moisturizer also to the intermediate layer (intermediate ply), the application amount of the moisturizer to an outer surface (exposed surface) of the outer layer sheet can be reduced, and occurrence of stickiness can be eliminated or suppressed.

It is common to crepe a ply base paper in order to impart flexibility to a tissue paper. Also in the embodiment, it is desirable to perform creping.

It is desirable that the number of crepes is 38 to 54 crepes/cm.

The number of crepes (crepes/cm) measured by one-shot 3D can be represented by a value obtained by drawing a linear shape in MD (Machine Direction) direction, counting the number of mountains having a mountain and valley shape of 1.0 to 2.0 cm in MD direction, and dividing the counted number by a measured length in MD direction.

When the shape is measured by one-shot 3D and a height profile on the X-Y plane is indicated on a screen, the height of a surface of the tissue paper is represented by a color tone. The shape of the crepe in the height direction can be confirmed from a measured cross-sectional curve profile. Here, since the crepe has mountains and valleys formed in a direction 90 perpendicular to MD direction, the number of crepes (crepes/cm) refers to a value obtained by counting the number of mountains having a mountain and valley shape of 1.0 to 2.0 cm in MD direction and dividing the counted number by a measured MD length. An average value of the measured values at five points is defined as a value for one sample.

It is desirable that the degree of sharpness of an irregular peak portion "arithmetic mean curvature Spc of a peak" is 2.8 to 3.5 (1/mm) (the larger the value is, the sharper a fine protrusion on a surface is).

By such creping, a surface roughness is made different between one surface and the other surface of the ply base paper.

When the moisturizer is applied to the ply base paper, the moisturizer is preferably applied to a surface having smaller irregularities due to creping.

That is, in a ply in which a surface roughness is expressed as a difference in magnitude of an arithmetic mean height Sa according to ISO 25718, it is desirable to apply the moisturizer to a surface having a smaller arithmetic mean height Sa.

When the moisturizer is applied to a surface having a smaller arithmetic mean height Sa, since the surface has a smaller arithmetic mean height Sa and contains a sufficient amount of the moisturizer, a smooth hand feel and softness are imparted.

Since the outer layer contains a chemical solution, when the stacked sheets are calendered after the moisturizer is applied to the outer layer, the paper is calendered by an external pressure applied to an outer surface of the outer layer, and the applied moisturizer uniformly diffuses to a surface. As a result, the surface is smooth, and more "surface smoothness" is exhibited.

In the tissue paper to which the moisturizer of the embodiment has been applied, it is desirable that the arithmetic mean height Sa of an outer surface (in particular, an exposed surface of the stacked sheets) under no pressure is 0.005 to 0.012 mm, particularly 0.006 to 0.008 mm. When the arithmetic mean height Sa is within the range, the surface is smooth and has moderate irregularities, and therefore wipeability of, for example, lipstick or foundation is excellent.

Here, "under no pressure" means that measurement is performed in a generated state.

Conversely, when the moisturizer is applied to a surface of the base paper ply having larger irregularities due to creping, the moisturizer is also hygroscopic, and therefore wrinkles are largely generated on the surface due to moisture absorption over time, the surface shrinks, and the surface smoothness is impaired.

The arithmetic mean height Sa is defined in ISO 25178, and represents an average of absolute values of differences in height between points with respect to an average surface of surface in a definition region. The smaller the numerical value is, the smaller the absolute value of the difference in height between points with respect to the average surface of surface is, and the flatter the surface is. The larger the numerical value is, the rougher the surface is.

Note that when the arithmetic mean height Sa is measured for a tissue paper product in which a tissue paper bundle is housed in a packaging body in a pop-up manner, a measurement surface of a sample taken out from the tissue paper bundle is a surface having a mountain side of a fold (the same applies to the arithmetic mean curvature Spc of a peak).

It is desirable that the tissue paper according to the embodiment has an arithmetic mean curvature (Spc) of 2.8 to 3.5 (1/mm) at a peak of an outer surface thereof under no pressure. The arithmetic mean curvature at a peak represents an arithmetic mean of principal curvatures at a peak in a defined region. The smaller the numerical value is, the more rounded a point in contact with another object is. The larger the numerical value is, the sharper a point in contact with another object is.

Note that a measurement surface of a sample obtained from the pop-up type bundle is a surface having a mountain side of a fold. When the arithmetic mean curvature (Spc) at a peak of an outer surface under no pressure is 2.8 to 3.5 (1/mm), wipeability of lipstick, foundation, or the like is excellent while a surface is felt to be smooth.

The "arithmetic mean height Sa" and "the arithmetic mean curvature (Spc) at a peak" in the present specification are values measured using a "one-shot 3D shape measuring machine VR-3200 (manufactured by KEYENCE CORPORATION)" (hereinafter, also referred to as "3D microscope") and its equivalent machine (non-contact three-dimensional measuring instrument).

The "3D microscope" can measure a shape of an object from a fringe projection image of an object projected from a monochrome C-MOS camera using structured illumination light emitted from a light projecting unit, and in particular, can measure the height, length, angle, volume, and the like of any part using the obtained fringe projection image. For observing, measuring, and analyzing an image obtained by the "3D microscope", software "VR-H2A" and its equivalent software can be used, respectively. Note that measurement is performed under conditions of a visual field area of 24 mm × 18 mm and a magnification of 12 times.

A specific procedure for measuring the arithmetic mean height Sa and the arithmetic mean curvature (Spc) at an outer surface under no pressure is as follows.

A tissue paper in a form of a ply, which is a sample (having a size of about 50 mm in MD direction and 50 mm in CD (Cross Direction) direction), is placed on a measuring table in a generated state while a measuring machine is in front of the tissue paper and a depth direction is MD direction. Note that a test piece used for the measurement is a flat portion of a product.

A screen of the software ("VR-H2A") is operated to obtain three images of a main image (texture), a main image (height), and a 3D image of a sample surface. Next, a "texture" image displayed, for example, by selecting "surface roughness" of the software is converted into a "height" image (an image represented by a shade of a color tone color-coded in a height direction).

Next, at least a maximum height (Sz), the arithmetic mean height Sa, and the arithmetic mean curvature (Spc) at a peak are set as measurement parameters, and measurement is performed. The size of the measurement range is 3.0 mm × 3.0 mm. With the above software, the measurement range can be set by selecting "designate numerical value" in "add region". The measurement range is set so as not to include an embossed portion, and the measurement range is set such that a shade of a color tone within each measurement range on a screen visually approaches a constant shade.

The measured values of the maximum height (Sz), the arithmetic mean height Sa, and the arithmetic mean curvature (Spc) at a peak are checked, and when the maximum height (Sz) exceeds 0.1000 mm, the value is discarded, and another measurement range is set. Note that the maximum height (Sz), the arithmetic mean height Sa, and the arithmetic mean curvature (Spc) at a peak are parameters of surface roughness defined in ISO 25178. In the measurement, in order to remove a large undulation of a sheet, the measurement is performed under conditions that a filter has a Galsian correction, an S-filter is not used, an F-operation has a two-dimensional curved surface, a 0.8 mm L-filter is used, and end point processing is ON.

Plane roughness is measured for the "maximum height (Sz)", the "mean arithmetic roughness (Sa)", and the "arithmetic mean curvature of peak (Spc) at a peak" in a range of 3.0 mm square as a measurement range. The plane roughness is measured in the range of 3.0 mm square in this image at a total of five places while the position is changed, and an average value at the five places is taken as a measurement value of each of the "maximum height (Sz)", the "mean arithmetic height (Sa)", and the "arithmetic mean curvature (Spc) at a peak" of the measurement sample. The range of 3.0 mm square, which is the measurement range, is located in a central portion with small distortion in a visual field area of 24 mm × 18 mm. Note that selection of each of the above five measurement ranges and the "maximum height (Sz)", the "mean arithmetic height (Sa)", and the "arithmetic mean curvature (Spc) at a peak" may be measured simultaneously, or may be measured while a range Z to be measured is changed.

The content of the moisturizer in each ply is 10.0 to 35.0%. In particular, it is desirable that the content is 17.0 to 30.0%. When the content is small, softness is not sufficient. When the content is large, stickiness occurs on an exposed surface, and a paper strength is easily reduced.

It is desirable that the tissue paper is a tissue paper having three plies stacked, the basis weight of one ply is 15.0 to 22.5 g/m², and the paper thickness of the three-ply paper is 140 to 270 µm, particularly 176 to 230 µm. In a multi-ply structure such as a three-ply paper, the paper thickness particularly affects "softness" and "fullness". In the tissue paper of the present embodiment, when the paper thickness is within this range, "softness", "fluffy bulkiness", and "surface smoothness" are remarkable.

When the tissue paper is a tissue paper including four stacked plies, it is desirable that the basis weight of one ply is 15.0 to 22.5 g/m², and the paper thickness of the four-ply paper is 180 to 360 µm, particularly 220 to 320 µm.

The paper thickness is a value obtained by sufficiently subjecting a test piece to humidity control under conditions of JIS P 8111 (1998), and then measuring the paper thickness using a dial thickness gauge (thickness measuring instrument) "PEACOCK G type" (manufactured by Ozaki MFG. Co. Ltd.) under the same conditions. Specifically, it is confirmed that there is no rubbish, dust, or the like between a plunger and a measuring table, the plunger is placed on the measuring table, a scale of the dial thickness gauge is moved to adjust a zero point, then the plunger is raised, a test piece is placed on the measuring table, the plunger is lowered slowly, and the current gauge is read. During the measurement, care is taken such that a terminal of the metallic plunger (a circular plane having a diameter of 10 mm) strikes against a paper plane perpendicularly. Note that a load is about 70 gf when the paper thickness is measured. The paper thickness is an average value of measured values obtained by performing this measurement 10 times at different sites. As the test piece, a three-ply product sheet is collected and measured while a fold line, a ply-bonded portion, and the like are avoided.

It is desirable that the tissue paper of the present embodiment has a dry strength of 220 to 420 cN/25 mm for three plies or four plies in a longitudinal direction. When the dry strength in the longitudinal direction is within this range, "softness", "fluffy bulkiness", and "surface smoothness" are remarkable. In addition, when the dry strength in the longitudinal direction is within this range, the strength is within a range of strength sufficient to withstand use.

When a ply base paper having excessively high dry strength for three plies in the longitudinal direction, that is, a ply base paper in which fibers are densely arranged in the longitudinal direction and compacted at a high pressure is used, softness is poor, and stickiness is likely to occur on an exposed surface because (it is predicted that) the moisturizer does not penetrate between the fibers.

It is desirable that the dry strength for three plies or four plies in a lateral direction is 60 to 160 cN/25 mm. When the dry strength in the lateral direction is within this range, "softness", "fluffy bulkiness", and "surface smoothness" are remarkable. In addition, when the dry strength in the lateral direction is within this range, the strength is within a range of strength sufficient to withstand use.

Furthermore, although not clear, a "dry tensile strength in the lateral direction" does not affect individual functionality such as "softness" or "fullness", but affects overall functionality of "skin texture". It has been found that there is a certain correlation between evaluation of the "skin texture" and the "dry strength in the lateral direction" when a subject freely touches a sample and then evaluates whether the tissue paper is good or bad on the basis of a comprehensive evaluation criterion of "skin texture" without evaluation based on a specific evaluation criterion of "softness" or "fullness".

It is desirable that the tissue paper of the present embodiment has a wet paper strength of 50 to 90 cN/25 mm for three plies or four plies in the lateral direction.

It is desirable that a ratio of wet tensile strength in the lateral direction/dry tensile strength in the lateral direction is 0.62 to 0.76. Note that this value is a measurement value with three plies or four plies as they are. With such a difference in strength, for example, when a user blows his or her nose, the user feels "robustness (strength and sense of security)" in a usage mode changing from a dry state to a wet state. Furthermore, it is difficult for the user to feel a change in strength of the paper in such a usage mode, which affects how to feel "smoothness" during use.

Note that the longitudinal direction of paper is also referred to as MD direction and is a flow direction during papermaking. The lateral direction of paper is also referred to as CD direction, and is a direction perpendicular to the flow direction (MD direction) during papermaking.

The dry (tensile) strength of the tissue paper of the embodiment is a value measured on the basis of JIS P 8113, and is a value measured as follows. As a test piece, a tissue paper cut into a size of about 25 mm (± 0.5 mm) (width) × about 150 mm (length) in both the longitudinal and lateral directions is used. A multi-ply tissue paper is measured with multiple plies. As a tester, a load cell tensile tester TG-200N manufactured by Minebea Co., Ltd. and its equivalent machine are used. Note that a grip interval is set to 100 mm, and a tensile speed is set to 100 mm/min. The measurement is performed by tightening both ends of the test piece to a grip of the tester, applying a tensile load to the paper piece in an up-down direction, and reading an indicated value (digital value) when the paper breaks. Five sets of samples are prepared in each of the longitudinal direction and the lateral direction, and each sample is measured five times. An average of the measured values is defined as a dry tensile strength in each of the directions. A sample is adjusted according to JIS P 8111 (1998).

The wet (tensile) strength of the tissue paper according to the embodiment is a value measured according to JIS P 8135 (1998), and is a value measured as follows. As a test piece, a tissue paper cut into a size of about 25 mm (± 0.5 mm) (width) × about 150 mm (length) in both the longitudinal and lateral directions is used. A multi-ply tissue paper is measured with multiple plies. As a tester, a load cell tensile tester TG-200N manufactured by Minebea Co., Ltd. and its equivalent machine are used. Note that a grip interval is set to 100 mm, and a tensile speed is set to 50 mm/min. The test piece used has been cured for 10 minutes in a dryer at 105°C. The measurement is performed by tightening both ends of the test piece to a grip of the tester, then horizontally adding water to a central portion of the test piece with a width of about 10 mm using a flat brush containing water, immediately thereafter applying a tensile load to the paper piece in an up-down direction, and reading an indicated value (digital value) when the paper breaks. Five sets of samples are prepared in each of the longitudinal direction and the lateral direction, and each sample is measured five times. An average of the measured values is defined as a wet tensile strength in each of the directions.

The dry tensile strength and the wet tensile strength can be adjusted by internally adding a dry paper strength enhancer or a wet paper strength enhancer to a paper material or wet paper. Examples of the dry paper strength enhancer include starch, polyacrylamide, carboxymethyl cellulose (CMC) or sodium carboxymethyl cellulose as a salt of CMC, calcium carboxymethyl cellulose, and zinc carboxymethyl cellulose. Examples of the wet paper strength enhancer include a polyamide polyamine epichlorohydrin resin, a urea resin, an acid colloid/melamine resin, and a thermally crosslinkable coating PAM. Note that when the dry paper strength enhancer is internally added, the addition amount thereof with respect to pulp slurry is about 1.0 kg/pulp t or less. The wet paper strength enhancer is desirably cationic, and the addition amount thereof with respect to pulp slurry is about 5.0 to 20.0 kg/pulp t.

A fiber material constituting the tissue paper is a pulp fiber, and it is desirable that the pulp fiber is needle bleached kraft pulp (NBKP) or leaf bleached kraft pulp (LBKP) used for a tissue paper. The tissue paper may contain waste paper pulp. However, it is extremely desirable that the tissue paper is constituted only by NBKP and LBKP which are each virgin pulp because it is difficult for the waste paper pulp to exhibit "softness". A blending ratio is NBKP : LBKP = 25 : 75 to 40 : 60 in terms of mass ratio. Within this range, it is possible for a user to remarkably feel "softness" and "smoothness" while feeling paper strength necessary for blowing his or her nose and "fluffy bulkiness".

It is desirable that a compression work amount is 1.85 to 2.50 gf/cm/cm², particularly 2.18 to 2.35 gf/cm/cm², and compression recoverability is 46.0 to 54.5%, particularly 48.0 to 53.0% for a total of 12 plies including four sets of three plies or a total of 12 plies including three sets of four plies.

### [Method for manufacturing tissue paper]

The tissue paper according to the present embodiment and a product obtained by bundling and packaging the tissue paper can be manufactured by the following manufacturing procedure.

First, a single-layer tissue paper base paper (ply base paper) having a crepe and formed by a papermaking unit is wound to form a primary original fabric roll. Next, the three (or four in a case of four plies) primary original fabric rolls are set in a stacking unit also referred to as a ply machine. A single continuous sheet is unwound from each of the primary original fabric rolls to be stacked in three layers (or four layers). Thereafter, for example, the stack is appropriately slit to manufacture a secondary original fabric roll.

Next, a stacked bundle is formed in a folding unit or the like also referred to as an interfolder using the secondary original fabric roll.

Thereafter, for example, the stacked bundle is cut into an appropriate size, and then packaged in a packaging body such as a paper packaging box or a plastic packaging bag to obtain a product related to the tissue paper.

In this series of manufacturing steps of forming a tissue paper into a product or between the manufacturing steps, a chemical agent adding unit is separately disposed, and a moisturizer (moisturizing chemical solution) containing glycerin is externally added to the tissue paper base paper.

Specifically, the moisturizing chemical solution may be added by incorporating a roll transfer device such as a flexographic printing machine or a gravure printing machine or a chemical solution application device such as a spray application device in either a ply machine or an interfolder, or by separating these devices from the ply machine or the interfolder.

In manufacturing the tissue paper of the embodiment, the moisturizer is added to each ply base paper. This form can be appropriately selected.

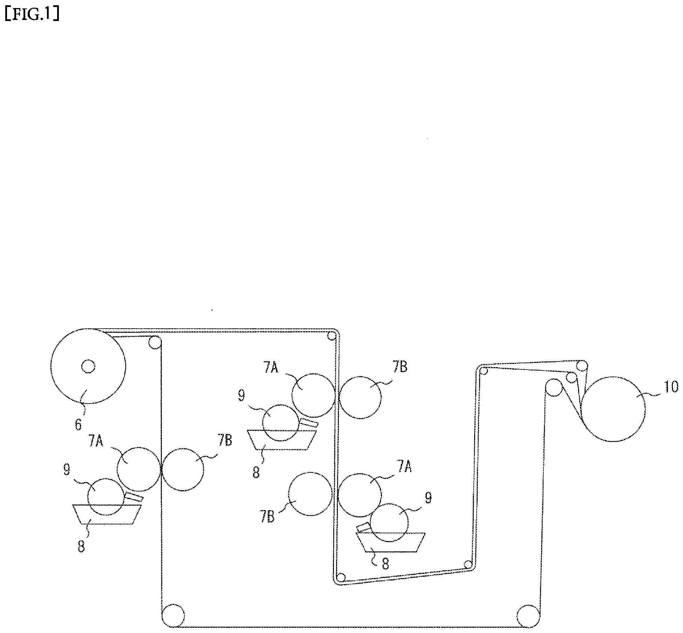

The moisturizing chemical solution application form can be, for example, the form of Fig. 1.

That is, a three-ply base paper is unwound from a three-ply base paper roll 6, a moisturizing chemical solution is picked up from a tray 8 by a gravure roll 9, the moisturizing chemical solution is transferred to a metal roll 7A, and the moisturizer is applied to an exposed surface of each of one outer layer and an intermediate layer while the moisturizing chemical solution passes between the metal roll 7A and another facing metal roll 7B.

At a subsequent position, the moisturizing chemical solution is applied to an outer surface of the other second outer layer of the three-ply base paper sheet in a similar form.

The sheet to which the moisturizing chemical solution has been applied is wound as an original fabric roll 10.

The moisturizing chemical solution may be applied by a flexographic method. However, it has been confirmed that a gravure application method has a higher effect of smoothing a surface than the flexographic method.

As another application form, for example, in an example of three plies, a first application unit in which a first pickup for picking up the moisturizer and a first transfer roll for receiving the first pickup roll are paired, and a second application unit in which a second pickup roll for picking up the moisturizer and a second transfer roll for receiving the second pickup roll are paired are disposed so as to face each other.

A first ply base paper as one outer layer and a second ply base paper as the other inner layer are supplied between the first transfer roll and the second transfer roll facing each other in a stacked state, and the moisturizer is applied to the outer surface of the first ply base paper and the outer surface of the second ply base paper from the first transfer roll and the second transfer roll, respectively.

A third application unit in which a third pickup roll for picking up the moisturizer and a third transfer roll for receiving the third pickup roll are paired is disposed at a subsequent stage of the pre-stage application, and the moisturizer is transferred to an outer surface of the third ply base paper as the outer layer on the opposite side by the third transfer roll and applied.

In this case, the moisturizer can be transferred to the outer surface of the third ply base paper by the third transfer roll and applied using the first transfer roll or the second transfer roll as a backup roll in addition to disposing the third transfer roll so as to face an appropriate backup roll.

For adjustment of an application amount to a target surface by each transfer roll, a pickup amount by each pickup roll can be adjusted by, for example, the degree of scraping by a blade. In addition, the application amount to a target surface by a transfer roll can also be adjusted by a rotational speed difference between the pickup roll formed by an anilox roll and the transfer roll.

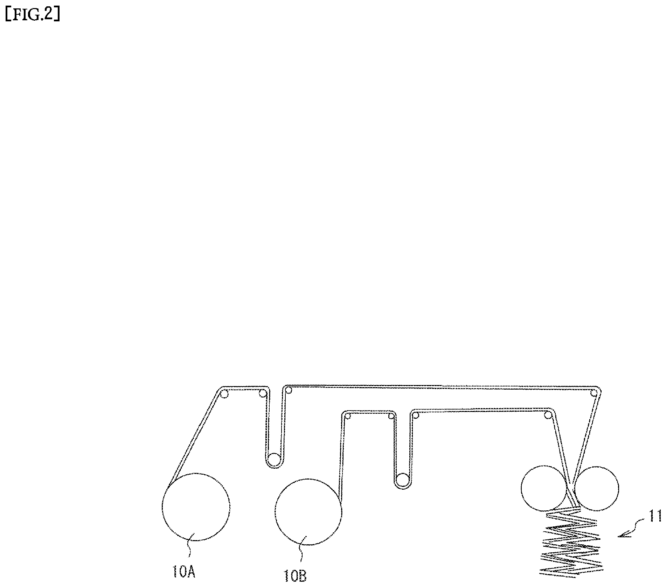

As illustrated in Fig. 2, a pair of stacked original fabric rolls 10A and 10B of the stacked sheets to which the moisturizing chemical solution has been applied is folded and formed into a product by, for example, a rotary interfolder 11.

The interfolder that performs folding may be a unit that performs folding using a folding plate also referred to as a multi-stand type, a stand type, or a folding plate type, or may be a unit that performs folding using a pair of folding rolls also referred to as a rotary type.

In the embodiment, it is desirable that a rotary interfolder is used. In a case of a tissue paper product having a multi-ply structure of three or more plies, the number of stacked layers is large, and deviation between the layers is likely to occur. However, in the rotary interfolder, for example, since a tension applied to a continuous sheet is weaker than that of another unit, deviation between the layers is less likely to occur, and a folding quality is likely to be good. Therefore, "fullness" is less likely to deteriorate particularly during processing.

Furthermore, it is desirable to perform calendering on the stacked sheets. By calendering the stacked sheets, a paper thickness difference between the outer layer and the intermediate layer is easily generated. In particular, when folding is performed by a rotary interfolder, it is desirable to add the moisturizing chemical solution in the interfolder. Furthermore, when a first calendering step is performed before the moisturizing chemical solution is added, and a second calendering step is performed after the moisturizing chemical solution is added, it is easy to obtain a tissue paper in which "softness" and "smoothness" are remarkably felt while "fluffy bulkiness" is felt.

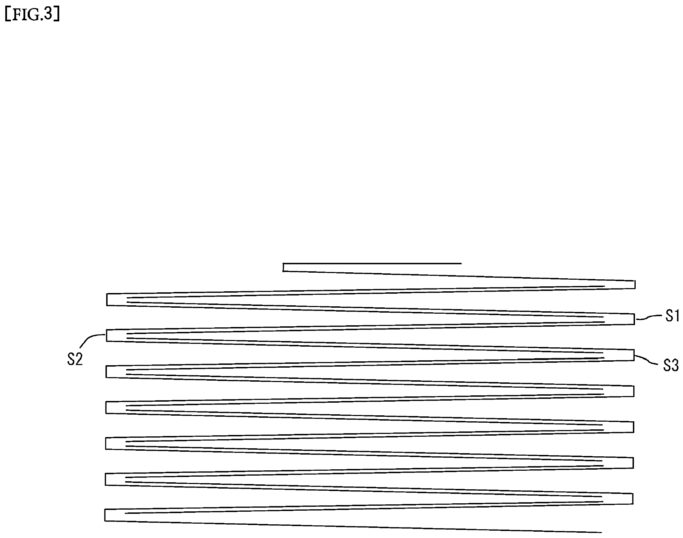

Fig. 3 illustrates a folded form example of the stacked sheets. This folded form example is a known form, in which one side of a preceding stacked sheet S1 and one side of a following stacked sheet S3 are stacked in a form of being inserted into a valley-folded portion of a stacked sheet S2, and as a result, the stacked sheets can be sequentially taken out from a packaging body (a paper packaging box, a plastic packaging bag, or the like) housing the stacked sheets in a pop-up manner.

A reason why "arithmetic mean roughness Sa" of a surface measured by one-shot 3D (manufactured by KEYENCE CORPORATION) and "compression work amount" and "compression recoverability" measured by KES-G5 (manufactured by KATO TECH CO., LTD.) are suitable for "tissue off" is roughly considered as follows.

A small value of the "arithmetic mean roughness Sa" imparts a smooth surface property.

A large value of "compression work amount" quickly starts compression and performs compression for a long time, which imparts good softness.

A small value of "compression recoverability" means that it is difficult to return to an original state once pushed, and therefore improves pressure controllability, which contributes to easy control of a level of force for wiping off lipstick or foundation.

### Examples

Samples of the tissue paper of the embodiment and a conventional tissue paper were prepared, and the following sensory tests were performed for items in the columns of the sensory tests as evaluation items. A physical property value, a composition value, and the like of each sample were measured as follows. A physical property value, a composition value, and a test result of each sample are as presented in Tables 1 and 2 for three plies, and as presented in Tables 3 and 4 for four plies.

### [Basis weight]

A basis weight was measured according to JIS P 8124 (1998).

### [Paper thickness]

As described above, a paper thickness was measured according to the above thickness measuring method using a dial thickness gauge (thickness measuring instrument) "PEACOCK G type" (manufactured by Ozaki MFG. Co. Ltd.) under conditions of JIS P 8111 (1998).

### [Dry tensile strength]

Already described.

### [Wet tensile strength]

A wet tensile strength was measured according to a tensile test of JIS P 8135 (1998).

As a test piece, a tissue paper cut into a size of about 25 mm (± 0.5 mm) (width) × about 150 mm (length) in both the longitudinal and lateral directions was used. A multi-ply tissue paper was measured with multiple plies as they were. As a tester, a load cell tensile tester TG-200N manufactured by Minebea Co., Ltd. was used. A grip interval was set to 100 mm. The measurement was performed by tightening both ends of the test piece which had been cured for 10 minutes with a dryer at 105°C to a grip of the tester, then horizontally adding water to a central portion of the test piece with a width of about 10 mm using a flat brush containing water, immediately thereafter applying a tensile load to the paper piece in an up-down direction, and reading a value (digital value) when the paper broke. A tensile speed was 50 mm/min. Five sets of samples were prepared in each of the longitudinal direction and the lateral direction, and each sample was measured five times. An average of the measured values was defined as a wet tensile strength in each of the directions.

### [Softness]

Softness was measured by a handle-o-meter method according to a JIS L 1096 E method. However, a test piece had a size of 100 mm × 100 mm, and measurement was performed with a clearance of 5 mm. Measurement was performed five times in each of a longitudinal direction and a lateral direction with a one-ply tissue paper, and an average value of all the ten values was represented in unit of cN/100 mm. Softness is one of indicators showing soft degree. Softness was measured with one ply. When a test piece is bonded by ply bonding or the like, the test piece is carefully peeled off, and softness is measured such that a ply-bonded portion is not located at the center of the test piece.

### [MMD]

While a contact surface of a friction element is brought into contact with a surface of a measurement sample to which a tension of 20 g/cm is applied in a predetermined direction at a contact pressure of 25 g, the measurement sample is moved by 2 cm in substantially the same direction as the direction in which the tension is applied at a speed of 0.1 cm/s, and a friction coefficient at this time is measured using a friction sense tester KES-SE (manufactured by Kato Tech Co., Ltd.). A value obtained by dividing the friction coefficient by a friction distance (moving distance = 2 cm) is MMD. The friction element is formed by adjoining 20 piano wires P each having a diameter of 0.5 mm, and has a contact surface formed such that the length and the width are both 10 mm. On the contact surface, a unit bulging portion having a tip formed with 20 piano wires P (radius of curvature: 0.25 mm) is formed.

Note that MMD is measured while a ply-bonded portion and a wrinkled portion are not included in advance.

On the other hand, the measurement value is an average value obtained by taking one arbitrary odd number set from three positions of an upper portion, a central portion, and a lower portion of the bundle of the stacked sheets for measurement. From a valley side of the three-layer (three-ply) sheet, the sheet is defined as a first layer (outer layer), a second layer (intermediate layer), and a third layer (outer layer).

Note 1 in Tables (Evaluation of chemical solution applied surface): Two test pieces cut into 2 cm square from each sheet were gently floated in water with a front surface side and a back surface side facing up on a spoon, and after 30 seconds, a side with less moisture on the test piece was defined as an applied surface (described as "CT"), and the other side was defined as a non-applied surface (described as "NCT"). When the applied surface is not clear, it is described as "-".

Note 2 in Tables is a value obtained by plane roughness measurement measured by one-shot 3D.

### [Sensory test]

Evaluation for a result of Comparative Example 2 was set to "4.0", and a result is indicated by an average value of scores of score 1 to score 7 by 12 evaluators on the basis of the result of Comparative Example 2. The higher the score, the higher the evaluation.

Comparative Examples 1 to 4 are commercially available products in China. Reference Example is a prototype by the applicant.

**[Table 1]**

| Item | | | Unit | Example 1 | Comparative Example 1 | Comparative Example 2 | Comparative Example 3 |
|---|---|---|---|---|---|---|---|
| Basis weight (one ply) | | | g/m² | 17.8 | 17.7 | 16.2 | 18.1 |
| Paper thickness (product) | | | µm | 211 | 236 | 200 | 163 |
| Number of plies | | | - | 3 | 3 | 3 | 3 |
| Dry strength (longitudinal) | | | cN/25 mm | 329 | 343 | 579 | 869 |
| Dry strength (lateral) | | | cN/25 mm | 113 | 170 | 120 | 128 |
| Wet strength (lateral) | | | cN/25 mm | 74 | 87 | 62 | 75 |
| Softness (one ply) Average of values for layers | | | cN/100 mm | 1.02 | 1.20 | 0.90 | 1.10 |
| MMD | First layer valley-folded surface and Third layer mountain-folded surface (-) | | | 7.5 | 6.9 | 8.2 | 6.6 |
| Moisture percentage | | | % | 13.0 | 13.2 | 13.5 | 16.1 |
| Dry chemical solution content percentage of each layer when dry chemical solution content percentage of product is 100 | First layer | Outer layer | % | 99.4 | 103.5 | 104.3 | 106.2 |
| | Second layer | Intermediate layer | % | 100.8 | 90.1 | 90.9 | 89.2 |
| | Third layer | Outer layer | % | 99.8 | 106.3 | 104.7 | 104.6 |
| Dry chemical solution content percentage of product | | | % | 22.0 | 21.6 | 23.2 | 25.3 |
| Chemical solution application percentage (with respect to mass of base paper) | | | % | 28.0 | 27.5 | - | - |

**[Table 2]**

| | | | | | | | | |
|---|---|---|---|---|---|---|---|---|
| Evaluation of applied surface/non-applied surface *Note 1 | First layer | Valley-folded surface | | | CT | CT | CT | CT |
| | | Mountain-folded surface | | | NCT | NCT | NCT | NCT |
| | Second layer | Valley-folded surface | | | NCT | - | - | - |
| | | Mountain-folded surface | | | CT | - | - | - |
| | Third layer | Valley-folded surface | | | NCT | NCT | NCT | NCT |
| | | Mountain-folded surface | | | CT | CT | CT | CT |
| Sa Arithmetic mean height *Note 2 | First layer | Valley-folded surface | | mm | 0.0065 | 0.0063 | 0.0045 | 0.0044 |
| | | Mountain-folded surface | | mm | 0.0070 | 0.0064 | 0.0049 | 0.0049 |
| | Second layer | Valley-folded surface | | mm | 0.0069 | 0.0061 | 0.0038 | 0.0039 |
| | | Mountain-folded surface | | mm | 0.0065 | 0.0065 | 0.0040 | 0.0042 |
| | Third layer | Valley-folded surface | | mm | 0.0068 | 0.0061 | 0.0045 | 0.0046 |
| | | Mountain-folded surface | | mm | 0.0064 | 0.0061 | 0.0040 | 0.0043 |
| Compression property KES-G% 4 sets of 3 plies | Compression work amount | | gf/cm/cm² | | 2.28 | 2.15 | 1.71 | 1.36 |
| | Compression recoverability | | % | | 50.6 | 53.6 | 55.4 | 57.5 |
| | | | | | | | | |
| Sensory evaluation | Softness | | 1 to 7 | | 5.4 | 5.1 | 4.0 | 4.1 |
| | Smoothness | | 1 to 7 | | 5.2 | 4.7 | 4.0 | 3.8 |
| | Sticky feeling of surface | | 1 to 7 | | 5.5 | 4.4 | 4.0 | 3.0 |
| | Thick feeling | | 1 to 7 | | 4. 9 | 4.8 | 4.0 | 4.1 |

**[Table 3]**

| Item | | | | Unit | Example 2 | Comparative Example 4 |
|---|---|---|---|---|---|---|
| Basis weight (one ply) | | | | g/m² | 16.5 | 16.2 |
| Paper thickness (product) | | | | µm | 271 | 266 |
| Number of plies | | | | - | 4 | 4 |
| Dry strength (longitudinal) | | | | cN/25 mm | 356 | 380 |
| Dry strength (lateral) | | | | cN/25 mm | 114 | 122 |
| Wet strength (lateral) | | | | cN/25 mm | 83 | 88 |
| Softness (one ply) Average of values for layers | | | | cN/100 mm | 0.99 | 1.14 |
| MMD | | First layer valley-folded surface and Fourth layer mountain-folded surface (-) | | | 6.9 | 7.3 |
| Moisture percentage | | | | % | 13.6 | 12.9 |
| Dry chemical solution content percentage of each layer when dry chemical solution content percentage of product is 100 | First layer | | Outer layer | % | 99.8 | 111.8 |
| | Second layer | | First intermediate layer | % | 102.1 | 88.9 |
| | Third layer | | Second intermediate layer | % | 98.0 | 87.2 |
| | Fourth layer | | Outer layer | % | 99.2 | 113.3 |
| Dry chemical solution content percentage of product | | | | % | 21.9 | 21.6 |
| Chemical solution application percentage (with respect to mass of base paper) | | | | % | 28.3 | 27.8 |

**[Table 4]**

| | | | | | |
|---|---|---|---|---|---|
| Evaluation of applied surface/non-applied surface *Note 1 | First layer | Valley-folded surface Mountain-folded surface | | CT NCT | CT NCT |
| | Second layer | Valley-folded surface Mountain-folded surface | | NCT CT | - - |
| | Third layer | Valley-folded surface Mountain-folded surface | | NCT CT | - - |
| | Fourth layer | Valley-folded surface Mountain-folded surface | | NCT CT | NCT CT |
| Sa Arithmetic mean height *Note 2 | First layer | Valley-folded surface | mm | 0.0064 | 0.0062 |
| | | Mountain-folded surface | mm | 0.0070 | 0.0070 |
| | Second layer | Valley-folded surface | mm | 0.0071 | 0.0062 |
| | | Mountain-folded surface | mm | 0.0066 | 0.0065 |
| | Third layer | Valley-folded surface | mm | 0.0063 | 0.0062 |
| | | Mountain-folded surface | mm | 0.0066 | 0.0069 |
| | Fourth layer | Valley-folded surface | mm | 0.0070 | 0.0065 |
| | | Mountain-folded surface | mm | 0.0063 | 0.0060 |
| Compression property KES-G% 3 sets of 4 plies | Compression work amount | | gf/cm/cm² | 2.24 | 2.10 |
| | Compression recoverability | | % | 49.8 | 53.9 |
| | | | | | |
| Sensory evaluation | Softness | | 1 to 7 | 5.2 | 4. 9 |
| | Smoothness | | 1 to 7 | 5.2 | 4.5 |
| | Sticky feeling of surface | | 1 to 7 | 5.7 | 4.4 |
| | Thick feeling | | 1 to 7 | 5.3 | 4.8 |

According to the above result, it is possible to obtain a three-ply or four-ply tissue paper that reduces a sticky feeling of an outer surface while securing necessary softness.

In the above experimental example, the evaluation of the chemical solution applied surface is simply performed, but more specifically, it has been found that it is desirable to perform the evaluation as follows.
(1) A pan containing 500 mL of water, a spoon, and a sample are prepared.
(2) A three-layer or four-layer stacked sheet is cut into 2 cm × 2 cm excluding embossed and wrinkled portions, and one piece thereof is divided into the sheets (three sheets or four sheets). Two sets are prepared as a test piece A and a test piece B.
(3) A surface A (test piece A) is defined from a valley side surface of each divided sheet, and a back surface thereof is defined as a surface B (test piece B). The surface A is marked with a red ballpoint pen.
(4) On a bowl of a chemical agent spoon (about 20 cm in length), for each sheet, the test piece A with the surface A facing upward and the test piece B with the surface B facing upward are placed.
(5) The test piece A and the test piece B are gently floated on a water surface in the pan at the same time.
(6) After 30 seconds, a state of a water film on the test piece and a light reflection state are observed visually (by photograph).
(7) A surface (test piece) of the test piece where reflection of water is clearly large is defined as a non-applied surface (described as "NCT"). A surface (test piece) opposite to NCT is defined as an applied surface (described as "CT").
(8) When there is no apparent difference as determined by visual observation (photograph), both surfaces are described as "-".

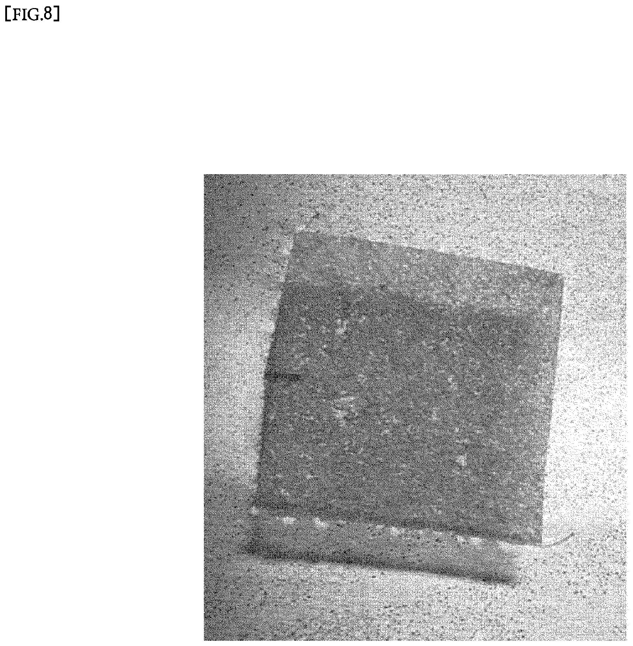

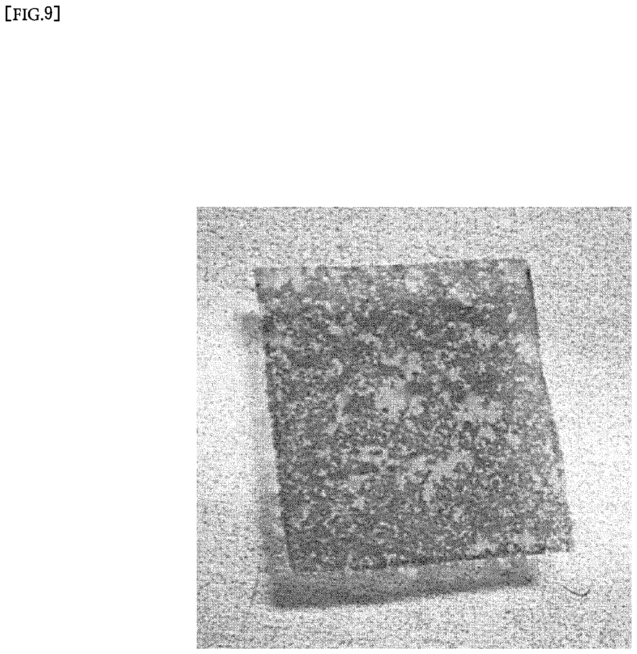

Test examples are illustrated in Figs. 8 and 9.

When the case of Fig. 8 for the test piece A is looked at, the surface viewed is the surface A = chemical solution applied surface, and the back surface is wetted, but there are few portions where water is exposed on the upper surface (surface A = chemical solution applied surface). It is considered that moisture that has passed through a gap (pore) of the paper from the bottom reaches the upper surface but is less likely to spread to the upper surface because the surface tension of the chemical solution is low.

On the other hand, when the case of Fig. 9 for the test piece B is looked at, the surface viewed is the surface B = chemical solution not applied surface, the back surface is wetted, and there are many portions where water is exposed on the upper surface (surface B = chemical solution not applied surface). It is considered that since the amount of the chemical solution on the upper surface is relatively small, moisture that has passed through a gap (pore) of the paper from the bottom has a high surface tension and easily spreads to the upper surface.

As for "chemical solution content", the chemical solution is contained in an amount of 10.0 to 35.0% by mass in each sheet moisture-controlled at 23°C and 50% RH in the standard state of JIS P 8111. For measurement of the content of the chemical solution, each sheet of a chemical solution applied multilayer sanitary tissue paper is peeled off, the weight (a) thereof is measured in the standard state of JIS P 8111, a test piece is put in a solvent in which a ratio of ethyl alcohol : acetone is set to 50 : 50 by a Soxhlet extractor, and a chemical solution applied to the chemical solution applied multilayer sanitary tissue paper is eluted while the solvent is lightly boiled for about three hours. The test piece is taken out, dried at 60°C until a constant amount is reached, and the weight (b) is measured. "Chemical solution content (%)" is calculated by {(a) - (b)} ÷ (a) × 100. This means the mass ratio of the dried chemical solution contained in the product.

### Reference Signs List

- 6: Base paper roll
- 9: Gravure roll
- 10: Original fabric roll
- 10A, 10B: Stacked original fabric roll
- 11: Rotary interfolder
- 1R to 4R: Ply
- F: Applied surface
- W: Non-applied surface

## Claims

1. A tissue paper comprising three stacked plies or four stacked plies,
wherein each ply contains an aqueous moisturizer exhibiting hygroscopicity, and
a content of the aqueous moisturizer between the plies is set such that a dry moisturizer content percentage of each ply is within a range of 92.0 to 108.0 when a dry moisturizer content percentage of a product is 100.

2. The tissue paper according to claim 1,
wherein each ply has a difference in magnitude of an arithmetic mean height Sa according to ISO 25718 between both surfaces of the ply, and an outer surface of each ply located on an outer side in the three-ply or four-ply stack is a surface having a smaller arithmetic mean height Sa.

3. The tissue paper according to claim 1,
wherein an outer surface of each ply located on an outer side in the three stacked plies or four stacked plies has a higher content of the moisturizer than an inner surface of the ply.

4. The tissue paper according to claim 1,
wherein the moisturizer contains glycerin as a main component.

5. The tissue paper according to claim 1,
wherein a content of the moisturizer in each ply is 10.0 to 35.0%.

6. The tissue paper according to claim 1, comprising three stacked plies,
wherein one ply has a basis weight of 15.0 to 22.5 g/m², and the three-ply paper has a paper thickness of 140 to 270 µm.

7. The tissue paper according to claim 1, comprising four stacked plies,
wherein one ply has a basis weight of 15.0 to 22.5 g/m², and the four-ply paper has a paper thickness of 180 to 360 µm.

8. The tissue paper according to claim 1,
wherein an arithmetic mean height Sa according to ISO 25718 at each surface of each ply is 0.005 to 0.012 mm.

9. A tissue paper product comprising:
a housing; and
a tissue paper including three stacked plies or four stacked plies, the tissue paper being housed in the housing,
wherein each ply contains an aqueous moisturizer exhibiting hygroscopicity, and
a content of the aqueous moisturizer between the plies is within a range of 92.0 to 108.0 when an average value of contents in the plies is 100.

10. The tissue paper product according to claim 9,
wherein a compression work amount is 1.85 to 2.50 gf/cm/cm², and compression recoverability is 46.0 to 54.5% for a total of 12 plies including four sets of three plies or a total of 12 plies including three sets of four plies.
